# EUROPÄISCHE PATENTSCHRIFT

(11) **EP 3 679 876 B1**
(45) Veröffentlichungstag und Bekanntmachung des Hinweises auf die Patenterteilung: **30.07.2025**
(21) Anmeldenummer: 19218812.6
(22) Anmeldetag: 20.12.2019
(51) Int. Cl.: A61B 17/29, A61B 17/3201

(54) **IN DER CHIRURGIE VERWENDETES INSTRUMENT UND SPÜLEN DES INSTRUMENTS**
INSTRUMENT USED IN SURGERY AND FLUSHING OF THE INSTRUMENT
INSTRUMENT UTILISÉ EN CHIRURGIE ET RINÇAGE DE L'INSTRUMENT

(30) Priorität: 11.01.2019 DE 102019100601
(43) Veröffentlichungstag der Anmeldung: 15.07.2020
(73) Patentinhaber: Karl Storz SE & Co. KG, 78532 Tuttlingen (DE)
(72) Erfinder: Thouément, Yann, 78690 Les Essarts-Le-Roi (FR); Kärcher, Daniel, 78532 Tuttlingen (DE); Besse, Régis, 78610 Le Perray en Yvelines (FR)

(56) Entgegenhaltungen:
- WO-A1-00/18521
- US-A- 4 576 650
- US-A- 5 408 991
- US-A1- 2007 112 377
- US-A1- 2009 287 113
- US-A1- 2017 332 891

## Beschreibung

Die vorliegende Erfindung betrifft ein in der Chirurgie verwendetes Instrument und insbesondere das Spülen des Inneren des Instruments.

Diese Instrumente kommen in vielen Sparten der Chirurgie wie der Endoskopie, der Laparoskopie und der Neurochirurgie zum Einsatz.

Gehirntumore sind in den letzten Jahren chirurgisch durch Endoskopie (Neuroendoskopie) entfernt worden. In solchen Fällen erfolgt Gehirnchirurgie durch das Herstellen einer sehr kleinen Schädelöffnung. Ein an einem Endoskop angebrachtes Instrument wird in diese kleine Öffnung eingeführt, und die Operation wird durchgeführt. Das Instrument umfasst eine Zange und/oder eine Schere am distalen Ende. Einige Tumorarten wie der Hypophysentumor können durch Einführen eines an einem Endoskop angebrachten Instruments durch die Nase entfernt werden.

Während dieser Operation wird das Instrument zur Entfernung des Tumors eingesetzt, und daher dringen Blut, Gewebe und andere Verunreinigungen in den inneren Teil des Instruments ein. Daher ist der innere Teil des Instruments nach der Operation aufgrund von Blut, Gewebe und Verunreinigungen von der Operation in einem kontaminierten Zustand.

Dieses Instrument ist wiederverwendbar und es ist daher unbedingt nötig, den inneren Teil des Instruments nach der Operation gründlich entlang der gesamten Länge des Rohrs zu spülen.

Zum ordentlichen Spülen des Instrumentenrohrs wird ein Spülfluid durch den Fluidanschluss in das Instrumentenrohr eingeführt. Aufgrund der Position des Fluidanschlusses und der Länge des Instruments wird jedoch das distale Ende des Instrumentenrohrs manchmal nicht ordentlich gespült, und zum Spülen des distalen Teils des Rohrs ist mehr Aufwand nötig. In diesem Fall entweicht der größte Teil des Spülfluids durch den proximalen Teil des Instruments.

US 5 408 991 A beschreibt ein Endoskop mit einem oder mehreren Reinigungskanälen oder Lumen, die sich über mindestens einen Teil der Länge des Endoskops erstrecken.

Die Aufgabe dieser vorliegenden Erfindung besteht somit in der Bereitstellung einer Lösung für das Problem des Spülens des distalen Teils des Instrumentenrohrs.

Diese Aufgabe wird durch Bereitstellen eines zur Durchführung einer Operation ausgestalteten Instruments gelöst, das Folgendes umfasst:
einen distalen Endabschnitt,
einen proximalen Endabschnitt,
ein den distalen Endabschnitt und den proximalen Endabschnitt verbindendes Rohr
und einen Fluidanschluss, der einen Einlass zum Eintritt von Spülfluid in das Innere des Instruments und zwei zum Führen des Spülfluidflusses im Instrument ausgestaltete Auslässe umfasst,
wobei ein erster Auslass dazu ausgestaltet ist, dass er dem Spülfluid gestattet, zum distalen Ende des Instruments hin zu fließen und ein zweiter Auslass dazu ausgestaltet ist, dass er dem Spülfluid gestattet, zum proximalen Ende des Instrumentenrohrs hin zu fließen, dadurch gekennzeichnet, dass der erste Auslass größer als der zweite Auslass ist, wobei eine Schubstange vorgesehen ist, die vom proximalen Endabschnitt zum distalen Endabschnitt des Instruments verläuft und Spülfluid fließt im Instrumentenrohr um die Schubstange herum und umspült das Äußere der Schubstange wobei im proximalen Teil der Schubstange eine Feder vorgesehen ist, um die Schubstange während einer Operation zu betätigen und das Spülfluid umspült auch diese Feder.

Der größere Auslass, der es Spülfluid gestattet, zum distalen Ende des Instrumentenrohrs zu fließen, dass im Vergleich zum proximalen Teil mehr Spülfluid in der Richtung des distalen Teils fließt. Außerdem ist das Instrument so konstruiert, dass das Instrumentenrohr zur leichten Betätigung bei einer Operation an der distalen Seite des Instruments gebogen ist.

Ausführungsbeispiele der Erfindung gehen im Einzelnen aus der Zeichnung hervor und werden ausführlicher in der folgenden Beschreibung erläutert. Es zeigen:
- Fig. 1: Ansicht des gesamten Instruments von oben,
- Fig. 2: Ansicht des mit dem Instrument verbundenen Fluidanschlusses von oben,
- Fig. 3: Schnittansicht des Instruments und des Fluidanschlusses,
- Fig. 4: isometrische Schnittansicht des Instruments und des Fluidanschlusses.

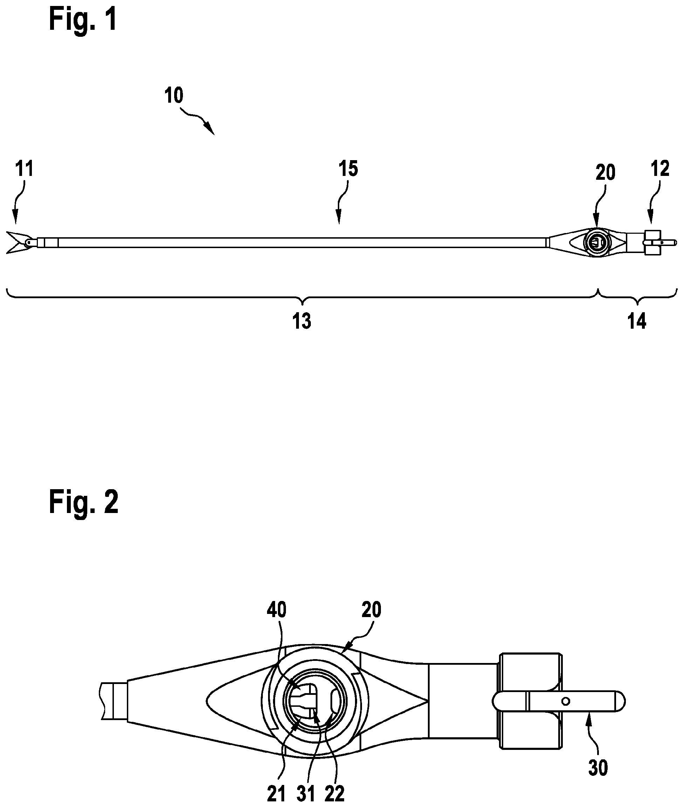

Fig. 1 zeigt die Ansicht des gesamten Instruments 10 von oben. Das Instrument umfasst einen proximalen Endabschnitt 12 und einen distalen Endabschnitt 11. Zwischen dem proximalen Endabschnitt und dem distalen Endabschnitt ist ein Rohr 15 angebracht. Am distalen Ende 11 ist zur Durchführung der Operation eine Zange oder Schere angebracht. Das proximale Ende des Instruments 12 kann zur Durchführung einer Operation an einem Griff (in der Fig. nicht gezeigt) angebracht werden.

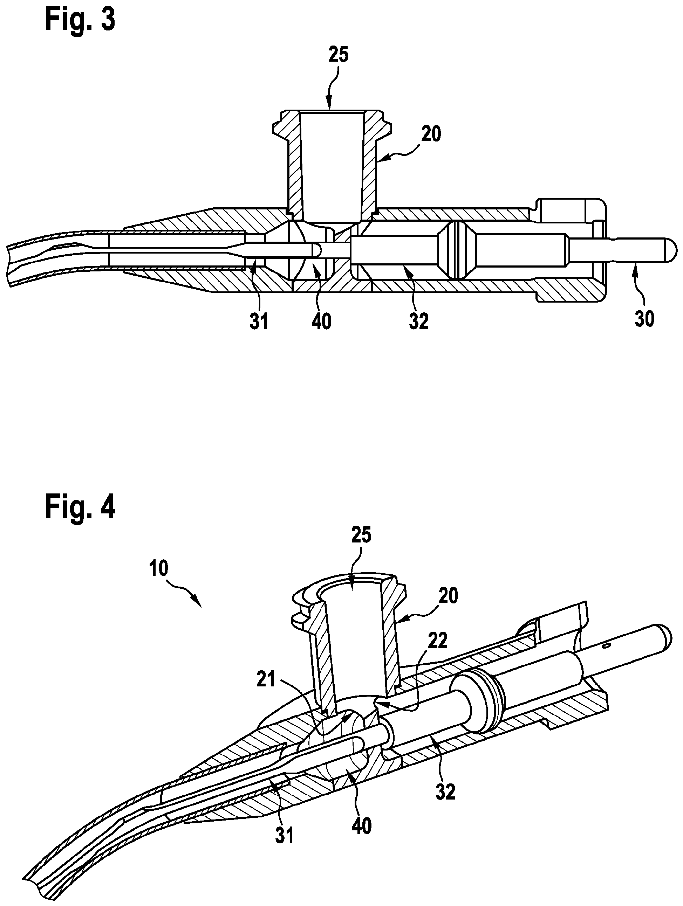

Der Fluidanschluss 20 ist mit dem proximalen Teil des Instruments in der Nähe des proximalen Endabschnitts verbunden. Figur 3 zeigt die Nahansicht des Fluidanschlusses, und der Aufbau des Fluidanschlusses wird anhand von Figuren 3, 4 und 5 beschrieben.

Durch den Fluidanschluss eingespritztes Spülfluid fließt zum proximalen und distalen Teil des Instruments. Aus der Figur wird deutlich, dass der distale Teil 13 des Instruments, dessen Inneres gespült werden muss, länger als der proximale Teil 14 des Instruments ist, dessen Inneres gespült werden muss. Daher ist es schwierig, den gesamten distalen Teil 13 des Instruments zu spülen.

Fig. 2 zeigt die Nahansicht des Fluidanschlusses und der proximalen Seite des Instruments von oben. Wie in der Figur gezeigt, sind am unteren Teil des Fluidanschlusses zwei Auslässe 21 und 22 vorgesehen. Von diesen Auslässen fließt Spülfluid in zwei entgegengesetzten Richtungen. Der erste Auslass 21 ist so vorgesehen, dass Spülfluid in die Richtung des distalen Teils des Instrumentenrohrs fließt. Der zweite Auslass 22 ist so vorgesehen, dass Spülfluid in die proximale Seite des Instruments fließt. Wie aus der Figur deutlich hervorgeht, ist der erste Auslass 21, der es gestattet, dass das Spülfluid zum distalen Ende des Instruments fließt, er ist größer als der zweite Auslass 22, der es gestattet, dass das Spülfluid zum proximalen Ende des Instruments fließt.

Aufgrund der unterschiedlichen Größe beider Auslässe fließt im Vergleich zu dem Spülfluid, das zum proximalen Endabschnitt des Instruments fließt, mehr Spülfluid zum distalen Ende des Instrumentenrohrs. Dadurch herrscht am distalen Teil des Instruments höherer Druck. Dieser Druckaufbau im distalen Teil des Instruments unterstützt das Fließen des Spülfluids zum distalen Endabschnitt des Instrumentenrohrs. Dadurch wird das Spülen der gesamten Länge des Inneren des Instrumentenrohrs veranlasst.

Fig. 3 zeigt die zweidimensionale Schnittansicht des Instruments und insbesondere des Fluidanschlusses. Der Fluidanschluss ist an der proximalen Seite in der Nähe des proximalen Endes mit dem Instrument verbunden. Zum Spülen des Instrumentenrohrs nach einer Operation wird Spülfluid vom Einlass des Fluidanschlusses 25 eingeführt. Da an der Ausgabe des Fluidanschlusses zwei Auslässe 21, 22 vorgesehen sind, fließt ein Teil des Spülfluids durch den ersten Auslass 21 zum distalen Ende des Instruments und ein Teil des Spülfluids durch den zweiten Auslass 22 zum proximalen Ende des Instruments. Dieses Spülfluid fließt dann durch das Instrumentenrohr 40 und spült das Innere des Rohrs. Blut, Gewebe und Verunreinigungen, die sich im Instrumentenrohr während einer Operation angesammelt haben, werden durch dieses Spülfluid weggespült.

Es ist eine Schubstange 30 vorgesehen, die vom proximalen Endabschnitt zum distalen Endabschnitt des Instruments verläuft. Spülfluid fließt im Instrumentenrohr um die Schubstange herum und umspült das Äußere der Schubstange 30. Im proximalen Teil der Schubstange ist eine Feder 32 vorgesehen, um die Schubstange während einer Operation zu betätigen. Das Spülfluid umspült auch diese Feder.

Figur 4 zeigt eine isometrische Schnittansicht des Instruments und des Fluidanschlusses. Aus der isometrischen Ansicht wird die Konstruktion des Fluidanschlusses, der Schubstange und des Instrumentenrohrs besser deutlich. Wie gemäß Figuren 1 - 4 bereits beschrieben, umfasst das Instrument 10 einen Fluidanschluss 20, der mit der proximalen Seite des Instruments verbunden ist und der Fluidanschluss ist in zwei Auslässe unterteilt, so dass, wenn Spülfluid in den Einlass 25 des Fluidanschlusses eingeführt wird, der erste Auslass 21 es gestattet, dass das Spülfluid in die Richtung des distalen Endes fließt und der zweite Auslass 22 es gestattet, dass das Spülfluid in die Richtung der proximalen Seite fließt, so dass das Innere des Instrumentenrohrs 40 und das Äußere der Schubstange 30, 31, 32 gespült werden.

In der derzeitigen Ausführungsform der vorliegenden Erfindung wird zwar ein Fluidanschluss mit zwei Auslässen beschrieben, dank derer das Spülfluid am proximalen und distalen Teil des Instruments fließen kann, aber für den Fachmann ist es offensichtlich, dass am Fluidanschluss mehr als zwei Auslässe vorgesehen sein können, um nach Bedarf einen weiteren Teil des Instruments zu spülen.

### Liste von Bezugsziffern

- 10: Instrument
- 11: Distaler Endabschnitt des Instruments
- 12: Proximaler Endabschnitt des Instruments
- 13: Distaler Teil des Instruments
- 14: Proximaler Teil des Instruments
- 15: Instrumentenrohr
- 20: Fluidanschluss
- 21: Auslass des Fluidanschlusses, dank dessen Wasser am distalen Teil des Instruments fließen kann
- 22: Auslass des Fluidanschlusses, dank dessen Wasser am proximalen Teil des Instruments fließen kann
- 25: Fluideinlass in den Fluidanschluss
- 30: Schubstange
- 31: Schubstange an der distalen Seite des Instruments
- 32: Am proximalen Teil des Instruments vorgesehene Feder
- 40: Inneres des Instrumentenrohrs

## Patentansprüche

1. Instrument, das zur Durchführung einer Operation ausgestaltet ist, umfassend:
einen distalen Endabschnitt (11),
einen proximalen Endabschnitt (12),
ein den distalen Endabschnitt (12) und den proximalen Endabschnitt (11) verbindendes Rohr (15)
und einen Fluidanschluss (20), der einen Einlass (25) zum Eintritt von Spülfluid in das Innere des Instruments und mindestens zwei zum Führen des Spülfluidflusses im Instrument ausgestaltete Auslässe (21, 22) umfasst,
wobei der erste Auslass (21) dazu ausgestaltet ist, dass er dem Spülfluid gestattet, zum distalen Endabschnitt des Instruments hin zu fließen und der zweite Auslass (22) dazu ausgestaltet ist, dass er dem Spülfluid gestattet, zum proximalen Ende des Instrumentenrohrs (15) hin zu fließen,
**dadurch gekennzeichnet, dass** der erste Auslass (21) größer als der zweite Auslass (22) ist, wobei eine Schubstange (30) vorgesehen ist, die vom proximalen Endabschnitt zum distalen Endabschnitt des Instruments verläuft und Spülfluid fließt im Instrumentenrohr um die Schubstange herum und umspült das Äußere der Schubstange (30) wobei im proximalen Teil der Schubstange eine Feder (32) vorgesehen ist, um die Schubstange während einer Operation zu betätigen und das Spülfluid umspült auch diese Feder (32).

2. Instrument zur Verwendung in der Chirurgie nach Anspruch 1, umfassend
einen mit dem Instrument verbundenen Fluidanschluss, **dadurch gekennzeichnet, dass** der Fluidanschluss mit der proximalen Seite (14) des Instruments in der Nähe des proximalen Endabschnitts (12) des Instruments verbunden ist.

## Claims

1. An instrument configured to perform an operation, comprising:
a distal end section (11),
a proximal end section (12),
a tube (15) connecting the distal end section (12) and the proximal end section (11)
and a fluid port (20), which comprises an inlet (25) for the entry of flushing fluid into the interior of the instrument and at least two outlets (21, 22) configured to guide the flow of flushing fluid in the instrument,
wherein the first outlet (21) is configured to allow the flushing fluid to flow towards the distal end section of the instrument and the second outlet (22) is configured to allow the flushing fluid to flow towards the proximal end of the instrument tube (15),
**characterised in that** the first outlet (21) is larger than the second outlet (22), wherein a push rod (30) is provided which runs from the proximal end section to the distal end section of the instrument and flushing fluid flows in the instrument tube around the push rod and flushes around the exterior of the push rod (30), wherein a spring (32) is provided in the proximal part of the push rod to actuate the push rod during an operation and the flushing fluid also flushes around this spring (32).

2. The instrument for use in surgery according to claim 1, comprising
a fluid port connected to the instrument, **characterised in that** the fluid port is connected to the proximal side (14) of the instrument in the vicinity of the proximal end section (12) of the instrument.

## Revendications

1. Instrument, qui est conçu pour réaliser une opération, comprenant :
une partie d'extrémité distale (11),
une partie d'extrémité proximale (12),
un tube (15) reliant la partie d'extrémité distale (12) et la partie d'extrémité proximale (11)
et un raccord de fluide (20), qui comprend une admission (25) pour l'entrée de fluide de rinçage dans l'intérieur de l'instrument et au moins deux sorties (21, 22) conçues pour guider l'écoulement de fluide de rinçage dans l'instrument,
dans lequel la première sortie (21) est conçue pour être équipée pour permettre au fluide de rinçage de s'écouler vers la partie d'extrémité distale de l'instrument et la seconde sortie (22) est conçue pour être équipée pour permettre au fluide de rinçage de s'écouler vers l'extrémité proximale du tube (15) d'instrument,
**caractérisé en ce que** la première sortie (21) est plus grande que la seconde sortie (22), dans lequel une tige de poussée (30) est prévue, qui s'étend de la partie d'extrémité proximale à la partie d'extrémité distale de l'instrument et le fluide de rinçage s'écoule dans le tube d'instrument autour de la tige de poussée et rince l'extérieur de la tige de poussée (30), dans lequel un ressort (32) est prévu dans la partie proximale de la tige de poussée pour actionner la tige de poussée pendant une opération et le fluide d'irrigation rince également ce ressort (32).

2. Instrument destiné à être utilisé en chirurgie selon la revendication 1, comprenant
un raccord de fluide relié à l'instrument, **caractérisé en ce que** le raccord de fluide est relié au côté proximal (14) de l'instrument à proximité de la partie d'extrémité proximale (12) de l'instrument.
